(19) Europäisches Patentamt
European Patent Office
Office européen des brevets

(11) EP 0 498 178 B1

(12) **EUROPÄISCHE PATENTSCHRIFT**

(45) Veröffentlichungstag und Bekanntmachung des Hinweises auf die Patenterteilung:
**12.11.1997 Patentblatt 1997/46**

(51) Int Cl.[6]: **C07D 295/13**, C23F 11/14, E21B 41/02

(21) Anmeldenummer: **92100625.0**

(22) Anmeldetag: **16.01.1992**

(54) **Ammoniumsalze von Alkenylbernsteinsäurehalbamiden und ihre Verwendung als Korrosionsinhibitoren bei der Öl- und/oder Gasförderung**

Ammonium salts of alkenylsucciminic acids and their use as corrosionsinhibitors in gas- and oil production

Sels ammonium d'acides succinamiques et l'usage comme inhibiteurs de corrosion pendant la production de gaz et de pétrole

(84) Benannte Vertragsstaaten:
**DE FR GB IT NL**

(30) Priorität: **04.02.1991 DE 4103262**

(43) Veröffentlichungstag der Anmeldung:
**12.08.1992 Patentblatt 1992/33**

(73) Patentinhaber: **BASF Aktiengesellschaft
67063 Ludwigshafen (DE)**

(72) Erfinder:
- **Oppenlaender, Knut, Dr.
W-6700 Ludwigshafen (DE)**
- **Wegner, Brigitte, Dr.
W-6720 Speyer (DE)**
- **Slotman, Wilhelmus
W-6700 Ludwigshafen (DE)**

(56) Entgegenhaltungen:
**EP-A- 0 065 191**
**EP-A- 0 106 234**
**EP-A- 0 359 048**
**DE-A- 2 943 963**
**DE-A- 3 300 874**
**US-A- 4 557 838**

Bemerkungen:
Die Akte enthält technische Angaben, die nach dem Eingang der Anmeldung eingereicht wurden und die nicht in dieser Patentschrift enthalten sind.

EP 0 498 178 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Die Erfindung betrifft Ammoniumsalze von Alkenylbernsteinsäurehalbamiden und ihre Verwendung als Korrosionsinhibitoren bei der Öl- und/oder Gasförderung gegen die Korrosion durch Medien, die $CO_2$ und $H_2S$ sowie gegebenenfalls elementaren Schwefel enthalten.

Bei der Förderung von Erdöl und Erdgas entsteht meistens ein Öl- bzw. Gas-Wasser-Gemisch, welches im Falle von Erdöl bis zu ca. 98 % Wasser enthalten kann.

Der Salzgehalt des mitgeförderten Wassers kann äußerst gering sein, aber das Wasser kann auch salzgesättigt sein. Außerdem wird $H_2S$ und/oder $CO_2$ mitgefördert.

$H_2S$ und/oder $CO_2$ in Kombination mit dem Wasser verursachen starke Korrosionen an den in der Erdöl- und Erdgasindustrie verwendeten Metallen. Diese Korrosion wird noch verstärkt, wenn elementarer Schwefel anwesend ist.

Es ist bekannt und geübte Praxis, bei der Öl- und Gasförderung, dem Transport und der Lagerung durch Injizieren eine Lösung oder Dispersion von Korrosionsinhibitoren in das korrosive Medium einzubringen, so daß sich auf der Oberfläche der mit dem Medium in Berührung kommenden Metallteile eine Schutzschicht ausbildet.

Für diese Zwecke sind bisher bestimmte Imidazolinsalze vorgeschlagen worden, EP 0 065 191, EP 0 103 737), die jedoch gegen $CO_2/H_2S$-Korrosion insbesondere in Anwesenheit von elementarem Schwefel, wie auch in "Environment Treatment & Control", February 1990, S. 48 bis 52, beschrieben, keine befriedigende Korrosionsschutzwirkung besitzen.

Auch Aminsalze von Maleinamidsäuren, wie in EP 0 106 234 beschrieben, besitzen für diese Zwecke keine ausreichende Korrosionsschutzwirkung.

Öllösliche Amidoaminsalze von Alkenylbernsteinsäuren und ihre Verwendung als Korrosionsinhibitoren in Wasser-in-Öl-Emulsionen, wie sie bei der Erdölförderung auftreten, werden in der EP 0 359 048 beschrieben.

Wasserlösliche Alkanolaminsalze von Alkenylbernsteinsäuren, die in DE 29 43 963-C2 als Korrosionsinhibitoren bei der Metallbearbeitung beschrieben sind, zeigen in den hier untersuchten Systemen ebenfalls keine ausreichende Wirkung.

Aus der DE-A 33 00 874 sind Ammoniumsalze von Alkenylbernsteinsäurehalbamiden in Form ihrer Alkanolaminsalze als Korrosionsinhibitoren in wäßrigen Systemen bekannt.

Es bestand daher die Aufgabe, für korrosive Medien, die bei der Öl- und/oder Gasförderung auftreten, bessere, wenn möglich wasserlösliche Inhibitoren gegen die $CO_2/H_2S$-Korrosion, insbesondere bei Anwesenheit von elementarem Schwefel, zur Verfügung zu stellen.

Es wurde nun gefunden, daß Ammoniumsalze von Alkenylbernsteinsäurehalbamiden der folgenden Formel I

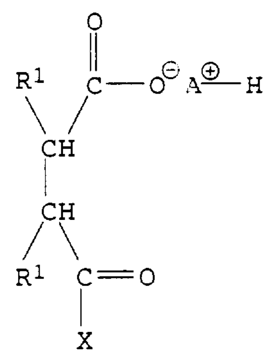

I

worin

$R^1$ H oder ein $C_6$-$C_{18}$-Alkenyl-Rest, mit der Maßgabe, daß die Reste $R^1$ weder beide H noch beide ein $C_6$-$C_{18}$-Alkenyl-Rest sind,

X eine Gruppe der allgemeinen Formel II

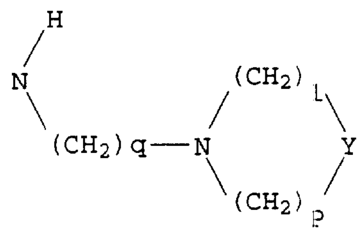

II

mit q = 1 bis 4, 1 und p unabhängig voneinander 1 bis 2 sowie Y = $CR^8_2$, $NR^8$ (mit $R^8$ jeweils H oder $C_1$-$C_3$-Alkyl), O oder S und

A ein Amin der Formel XH bedeuten,

als wasserlösliche Korrosionsinhibitoren bei der Öl- und/oder Gasförderung besonders gut geeignet sind.

Als besonders günstig hat sich als Gruppe X ein Rest der folgenden Formel III erwiesen:

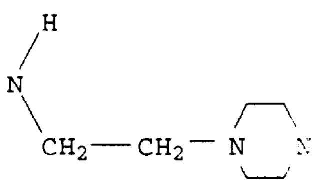

III

Die Gegenionen $A^{\oplus}$-H leiten sich von den zu Bildung der Halbamide eingesetzten Aminen XH ab.

Die den erfindungsgemäßen Salzen zugrundeliegenden Alkenylbernsteinsäureamide erhält man durch folgendes Verfahren:

1. An sich bekannte En-Reaktion von Maleinsäureanhydrid mit dem entsprechenden Olefin bei Temperaturen zwischen 150 und 250°C und Normaldruck oder bei 150 bis 300°C im Autoklaven (dieses Verfahren ist z.B. in der DE-A-34 11 531 erläutert) und

2. Umsetzung der so erhaltenen Alkenylbernsteinsäureanhydride mit den Aminen XH zu den Alkenylbernsteinsäurehalbamiden bei 50 bis 150°C, bevorzugt bei 50 bis 120°C, z.B. in Substanz oder in aromatischen Lösungsmitteln.

Die erfindungsgemäßen Amidammoniumsalze erhält man hieraus durch Reaktion mit den Aminen A, bevorzugt ebenfalls bei Temperaturen zwischen 50 und 120°C. Da für XH und A die gleiche Aminkomponente verwendet wird, kann die Herstellung des Amid/Ammoniumsalzes auch direkt durch Umsetzung des Alkenylbernsteinsäureanhydrids mit dem Amin im Verhältnis 1 : 2 erfolgen.

Als Vertreter der Amine XH sind insbesondere zu nennen Aminoalkylpiperazine und Aminoalkylmorpholine, wie zum Beispiel Aminoethylpiperazin.

Die so erhaltenen Inhibitoren bieten einen ausgezeichneten Schutz gegen $H_2S$ und $CO_2$-Korrosion, insbesondere auch in Anwesenheit von elementarem Schwefel.

Die Wirksamkeit der Inhibitoren speziell in Anwesenheit von elementarem Schwefel kann durch Formulierung der Produkte mit oberflächenaktiven Substanzen noch gesteigert werden.

Als Dispergiermittel können eingesetzt werden: Niedermolekulare oder polymere anionische Tenside und Dispergiermittel, insbesondere Alkylsulfonsäuren, Alkylarylsulfonsäuren sowie Arylsulfonsäuren und deren Salze.

Die erfindungsgemäßen Korrosionsinhibitoren werden je nach Zusammensetzung des korrosiven Mediums in Mengen von 3 bis 1000 ppm zugesetzt. Zur Verhinderung reiner $CO_2$- bzw. $CO_2$/$H_2S$-Korrosion werden vorzugsweise 5 bis 50 ppm, in Anwesenheit von elementaren Schwefel 100 bis 300 ppm der Inhibitorformulierung zugesetzt.

Die folgenden Beispiele erläutern die Erfindung.

1. Herstellung der Ammoniumsalze

Beispiel 1

Eine Reaktionsmischung aus äquimolaren Mengen Tetramerpropylen (463 g) und Maleinsäureanhydrid (269,5 g) wurde mit 2,3 g Phenothiazin versetzt und 25 bis 30 Stunden bei 180 bis 200°C gerührt. Anschließend wurden nicht umgesetzte Edukte bei 175°C im Hochvakuum abdestilliert.

342 g des so hergestellten Alkenylbernsteinsäureanhydrids wurden in 400 ml Xylol vorgelegt und auf 80°C erwärmt. Bei dieser Temperatur wurden 368 g Aminoethylpiperazin zugetropft. Man ließ noch 3 Stunden bei dieser Temperatur nachrühren und entfernte einen Teil des Lösungsmittels im Wasserstrahlvakuum. Ausbeute: 608 g schwarzes, viskoses Produkt mit 85 % Wirksubstanz.

Bei dem eingesetzten Tetramerpropylen handelt es sich um den $C_{12}$-Schnitt der Olefin-Oligomerisierung.

Zusammensetzung (nach GC/MS): 0,5 %, Cg, 5,8 % $C_{10}$, 20,7 % $C_{11}$, 70,9 % $C_{12}$, 2,1 % $C_{13}$.

Die Zusammensetzung sowie das Isomerenverhältnis können je nach Ansatz etwas variieren.

Aus dem IR-Spektrum wird deutlich, daß es sich bei dem Reaktionsprodukt aus dem Alkenylbernsteinsäureanhydrid und dem Aminoethylpiperazin um Ammoniumsalze der Alkenylbernsteinsäurehalbamide handelt, da die Reaktionsprodukte sowohl die charakteristischen Amid- als auch noch die -$CO_2$-Banden aufweisen. Daneben ist noch eine Imid-Bildung zu beobachten.

Charakteristische IR-Banden:

Amid: 1680 bis 1630 und 1570 bis 1515 $cm^{-1}$
COO-: 1610 bis 1550 $cm^{-1}$
Imid: ca. 1770 und ca. 1700 $cm^{-1}$

Die erfindungsgemäßen Ammoniumsalze können aus der Reaktionslösung in an sich bekannter Weise isoliert werden.

Beispiel 2

Im Autoklaven wurden 387 g Trimerbutylen (hergestellt durch Trimerisierung von n-Buten) und 225 g Maleinsäureanhydrid vorgelegt. Anschließend wurden 5 bar Stickstoff aufgepreßt und die Reaktionsmischung 10 Stunden bei 200°C gerührt.

595 g dieses Reaktionsproduktes wurden in 300 ml Xylol gelöst und auf 80°C erwärmt. Bei dieser Temperatur wurden 578 g Aminoethylpiperazin zugetropft. Nachdem die Reaktionsmischung 4 Stunden bei dieser Temperatur gerührt wurde, wurde das Lösungsmittel teilweise im Wasserstrahlvakuum entfernt und man erhielt den gewünschten Korrosionsinhibitor als dunkles, viskoses Öl.

Beispiel 3

Es wurde wie in Beispiel 2 gearbeitet, nur wurde anstelle des Trimerbutylens ein lineares $C_{12}$-Olefin verwendet.

Beispiel 4

Das Alkenylbernsteinsäureanhydrid wurde entsprechend Beispiel 2 aus Trimerbutylen und Maleinsäureanhydrid hergestellt. Nicht umgesetztes Olefin und Anhydrid wurden bei 100°C, 30 mbar entfernt.

198 g (0,75 Mol) dieses Produktes wurden in Xylol gelöst und die Lösung auf 100°C erwärmt. Bei dieser Temperatur wurden 97 g (0,75 Mol) Aminoethylpiperazin langsam zugetropft. Die Reaktionslösung wurde noch ca. 5 Stunden bei 90 bis 100°C gerührt. Anschließend wurde das Lösungsmittel entfernt und man erhielt 290 g schwarzes, viskoses Umsetzungsprodukt.

Zur Neutralisation der freien Carboxylgruppen wurden 101 g (0,2 Mol) dieses Produktes bei 80°C in Xylol mit 26 g (0,2 Mol) Diethylentriamin umgesetzt. Nachdem das Produkt am Rotationsverdampfer auf ca. 83 % aufkonzentriert wurde, erhielt man den erfindungsgemäßen Korrosionsinhibitor als dunkles, viskoses Öl.

2. Anwendungsbeispiele

Zur Prüfung der Inhibitorkompositionen wurde ein dynamischer Test (sog. "Wheel-test", s. auch EP-A 0 359 048) durchgeführt, eine gängige Methode zum Testen von Korrosionsinhibitoren für die Erdöl- und Erdgasförderung. Als Testcoupons wurden große Stahlbleche aus ST 37-Material verwendet, die vorher gründlich geschmirgelt, mit Toluol

entfettet und gewogen wurden. Die Coupons wurden danach in das korrosive Medium eingebracht und 16 Stunden bei 80°C einer mechanischen Bewegung (40 Upm mittels einer die Testgefäße drehenden Welle) unterzogen. Die Testbleche wurden anschließend mit einer inhibierten Säure gereinigt, entfettet und nach Trocknung zur Bestimmung des Gewichtsverlustes gewogen. Die Auswertung erfolgte im Vergleich zu einem Blindwert (Versuch ohne Inhibitorzusatz).

Aus den Ergebnissen der Wägungen wurde nach der folgenden Gleichung der prozentuale Korrosionsschutz Z berechnet:

$$Z = \frac{G_0 - G_1}{G_0} \times 100\ [\%]$$

wobei $G_0$ die Geschwindigkeit der Korrosion ohne Inhibitor und $G_1$ die Geschwindigkeit mit Inhibitor bedeuten.

Die Ergebnisse sind aus den folgenden Tabellen ersichtlich.

Tabelle 1

| Medium: 10 % Testbenzin, 90 % Salzwasser (3 % NaCl), gesättigt mit $CO_2$ und/oder $H_2S$ | | | | |
|---|---|---|---|---|
| Sättigung mit Dosierung: | $CO_2/CO_2$ | | $CO_2/H_2S$ | |
| | 3 ppm | 7,5 ppm | 3 ppm | 7,5 ppm |
| Produkt | [%] Korrosionsschutz Z | | | |
| Beispiel 1 | 46 | 78 | 68 | 79 |
| Beispiel 2 | 51 | 75 | 74 | 80 |
| Beispiel 3 | 61 | 64 | 76 | 81 |
| Beispiel 4 | | | 80 | 80 |

Tabelle 2

Medium:
ca. 6,5 ml Salzwasser, gesättigt mit $H_2S$
ca. 193,5 ml Salzwasser, gesättigt mit $CO_2$
0,5 g Schwefel gemahlen
Dosierung: 150 ppm Korrosionsinhibitor

| Produkt | Dispergiermittel | Korrosionsschutz Z [%] |
|---|---|---|
| Beispiel 1 | - | 50 |
| Beispiel 1 | 10 % oligomeres Arylsulfonat (Tamol 9401®) | 82 |
| Beispiel 1 | 15 % Alkylbenzolsulfonat (ALBS) | 78 |
| Beispiel 2 | 10 % oligomeres Arylsulfonat | 85 |
| Beispiel 2 | 15 % ALBS | 78 |
| Beispiel 3 | 15 % ALBS | 80 |
| Beispiel 3 | 10 % oligomeres Arylsulfonat | 75 |
| Beispiel 4 | 15 % ALBS | 80 |

Vergleichsbeispiele

| Medium:<br>ca. 6,5 ml Salzwasser, gesättigt mit $H_2S$<br>ca. 193,5 ml Salzwasser gesättigt mit $CO_2$<br>0,5 g Schwefel gemahlen<br>Dosierung: 150 ppm Korrosionsinhibitor | | | |
|---|---|---|---|
| Produkt | | Dispergiermittel | Korrosionsschutz Z [%] |
| 1. | Acetat eines langkettigen Imidazolins | - | 17 |
| 2. | Amido-Imidazolin plus dimeris./trimeris. Fettsäure | - | 33 |
| 3. | Amido-Imidazolin | - | 25 |
| 4. | wie 1. | 10 % oligomeres Arylsulfonat | 0 |
| 5. | wie 1. | 15 % Alkylbenzolsulfonat | 12 |

Aus diesen Ergebnissen geht hervor, daß mit bekannten, auf dem Markt erhältlichen Korrosionsinhibitoren eine deutlich schlechtere Wirkung erreicht wird und daß die Zugabe von Dispergiermitteln zu den bekannten Korrosionsinhibitoren keine vorteilhafte Wirkung zeigt.

Demgegenüber erweisen sich die erfindungsgemäpen Ammoniumsalze als sehr wirksame Korrosionsinhibitoren.

**Patentansprüche**

**1.** Ammoniumsalze von Alkenylbernsteinsäurehalbamiden der folgenden Formel I

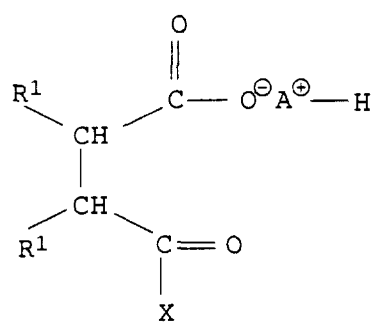

I

worin

$R^1$ H oder einen $C_6$-$C_{18}$-Alkenyl-Rest, mit der Maßgabe, daß die Reste $R^1$ weder beide H noch beide ein $C_6$-$C_{18}$-Alkenyl-Rest sind,

X eine Gruppe der allgemeinen Formel II

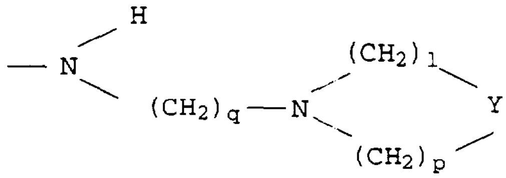

II

mit q = 1 bis 4, l und p unabhängig voneinander 1 bis 2 sowie Y = $CR^8{}_2$, $NR^8$ (mit $R^8$ jeweils H oder $C_1$-$C_3$-Alkyl), O oder S und

A ein Amin der Formel XH bedeuten.

**2.** Ammoniumsalze I mit einer Gruppe der allgemeinen Formel II für den Rest X nach Anspruch 1, wobei X eine Gruppe der Formel III

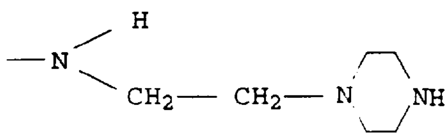

III

bedeutet.

**3.** Verfahren zur Herstellung von Ammcniumsalzen von Alkenylbernsteinsäurehalbamiden nach Anspruch 1, dadurch gekennzeichnet, daß man

a) in einer En-Reaktion $C_6$-$C_{18}$-Alkene mit Maleinsäureanhydrid bei 150 bis 250°C unter Normaldruck oder bei 150 bis 300°C im Autoklaven umsetzt,

b) die so erhaltenen Alkenylbernsteinsäureanhydride mit den Aminen XH bei 50 bis 150°C in 1 bis 8 Stunden zu Alkenylbernsteinsäurehalbamiden umsetzt und

die Alkenylbernsteinsäurehalbamide mit den Aminen A gemäß Anspruch 1 neutralisiert

**4.** Korrosionsschutzmittel, enthaltend ein Ammoniumsalz nach Anspruch 1 oder 2.

**5.** Korrosionsschutzmittel nach Anspruch 4, dadurch gekennzeichnet, daß es zusätzlich ein Dispergiermittel enthält.

**6.** Korrosionsschutzmittel nach Anspruch 5, dadurch gekennzeichnet, daß es als Dispergiermittel niedermolekulare oder polymere anionische Tenside, insbesondere Alkylsulfonsäuren, Alkylarylsulfonsäuren und/oder Arylsulfonsäuren und/oder deren jeweilige Salze enthält.

**7.** Verwendung von Ammoniumsalzen I mit einer Gruppe der Formel II oder III für den Rest X nach Anspruch 1 oder 2 als Korrosionsinhibitoren bei der Öl- und/oder Gasförderung.

**8.** Verwendung nach Anspruch 7, dadurch gekennzeichnet, daß das korrosive Medium $CO_2$ und/oder $H_2S$ enthält.

**9.** Verwendung nach Anspruch 8, dadurch gekennzeichnet, daß das korrosive Medium zusätzlich elementaren Schwefel enthält.

## Claims

**1.** An ammonium salt of an alkenylsuccinic acid semiamide of the following formula I

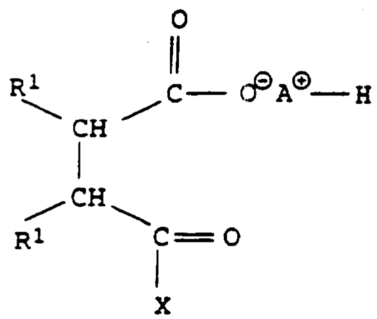

I

where

$R^1$ is H or $C_6$-$C_{18}$-alkenyl, with the proviso that the radicals $R^1$ are neither both H nor both $C_6$-$C_{18}$-alkenyl,
X is a group of the formula II

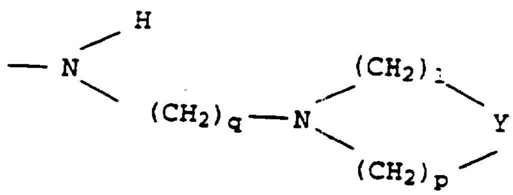

II

where q is from 1 to 4, l and p, independently of one another, are each 1 or 2 and Y is $CR^8_2$, $NR^8$ (where $R^8$ in each case is H or $C_1$-$C_3$-alkyl), O or S, and

A is an amine of the formula XH.

**2.** An ammonium salt I having a group of the formula II for the radical X as claimed in claim 1, X being a group of the formula III

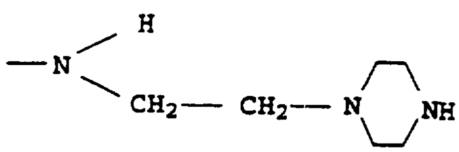

III

**3.** A process for the preparation of an ammonium salt of an alkenylsuccinic acid semiamide as claimed in claim 1, wherein

a) a $C_6$-$C_{18}$-alkene is subjected to an ene reaction with maleic anhydride at from 150 to 250°C under atmospheric pressure or at from 150 to 300°C in an autoclave,
b) the alkenylsuccinic anhydride thus obtained is reacted with an amine XH at from 50 to 150°C in the course of from 1 to 8 hours to give an alkenylsuccinic acid semiamide and
the alkenylsuccinic acid semiamide is neutralized with an amine A as claimed in claim 1.

**4.** A corrosion inhibitor containing an ammonium salt as claimed in claim 1 or 2.

**5.** A corrosion inhibitor as claimed in claim 4, which additionally contains a dispersant.

**6.** A corrosion inhibitor as claimed in claim 5, which contains, as the dispersant, a low molecular weight or polymeric anionic surfactant, in particular an alkanesulfonic acid, alkylarylsulfonic acid or arylsulfonic acid or a salt of one of these acids.

**7.** The use of an ammonium salt I having a group of the formula II or III for the radical X as claimed in claim 1 or 2 as a corrosion inhibitor in oil or gas production.

**8.** The use as claimed in claim 7, wherein the corrosive medium contains $CO_2$ or $H_2S$.

**9.** The use as claimed in claim 8, wherein the corrosive medium additionally contains elemental sulfur.

**Revendications**

**1.** Sels d'ammonium de monoamides d'acides alcénylsucciniques de formule générale I

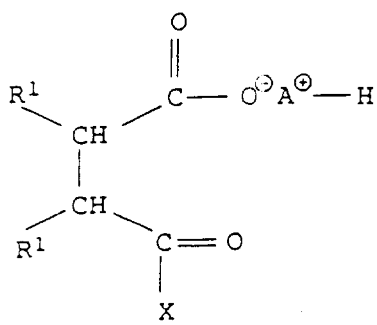

I

où

$R^1$ signifie H ou un reste alcényle en $C_6$-$C_{18}$, étant spécifié que les restes $R^1$ sont soit tous les deux H soit tous les deux un reste alcényle en $C_6$-$C_{18}$,

X signifie un groupement de formule générale II

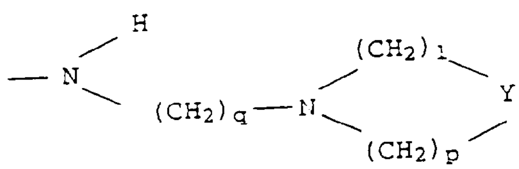

II

avec q = 1 à 4, l et p signifiant indépendamment l'un de l'autre 1 à 2 et Y = $CR^8_2$, $NR^8$ (avec $R^8$ étant à chaque fois H ou un groupement alkyle en $C_1$-$C_3$), O ou S et

A signifie une amine de formule XH.

**2.** Sels d'ammonium I dont le reste X est un groupement de formule générale II selon la revendication 1, où X représente un groupement de formule III

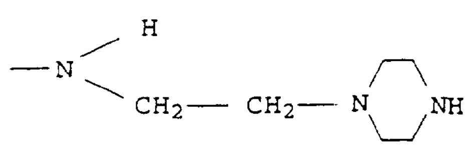

III

**3.** Procédé de préparation de se s d'ammonium de monoamides d'acides alcénylsucciniques selon la revendication 1, caractérisé en ce que

a) on fait réagir en une ène-réaction des alcènes en $C_6$-$C_{18}$ avec de l'anhydride maléique à 150-250°C sous pression normale ou à 150-300°C en autoclave,

b) on fait réagir les anhydrides d'acides alcénylsucciniques avec les amines XH à 50-150°C pendant 1-8 heures pour donner des monoamides d'acides alcénylsucciniques et
on neutralise les monoamides d'acides alcénylsucciniques avec les amines A selon la revendication 1.

**4.** Agent de protection contre la corrosion contenant un sel d'ammonium selon la revendication 1 ou 2.

**5.** Agent de protection contre la corrosion selon la revendication 4, caractérisé en ce qu'il contient en outre un agent dispersant.

**6.** Agent de protection contre la corrosion selon la revendication 5, caractérisé en ce qu'il contient des tensio-actifs anioniques de bas poids moléculaire ou polymères, en particulier des acides alkylsulfoniques, des acides alkylarylsulfoniques et/ou des acides arylsulfoniques et/ou leurs sels respectifs, en tant qu'agent dispersant.

**7.** Utilisation de sels d'ammonium I dont le reste X est un groupement de formule II ou III selon la reverdication 1 ou 2, en tant qu'inhibiteurs de corrosion lors de l'extraction du pétrole et/ou du gaz.

**8.** Utilisation selon la revendication 7, caractérisée en ce que le milieu corrosif contient du $CO_2$ et/ou du $H_2S$.

**9.** Utilisation selon la revendication 8, caractérisée en ce que le milieu corrosif contient en outre du soufre à l'état d'élément.